# EUROPEAN PATENT SPECIFICATION

(11) **EP 1 607 071 B1**
(45) Date of publication and mention of the grant of the patent: **08.08.2007**
(21) Application number: 05253692.7
(22) Date of filing: 15.06.2005
(51) Int. Cl.: A61F 5/00, A61B 17/12

(54) **A method of assembling an adjustable band**
Verfahren zur Herstellung eines justierbaren Bandes
Procédé d'assemblage d'un ruban réglable

(30) Priority: 16.06.2004 US 870349
(43) Date of publication of application: 21.12.2005
(73) Proprietor: ETHICON ENDO-SURGERY, INC., Cincinnati, Ohio 45242 (US)
(72) Inventor: Conlon, Sean P., Loveland Ohio 45140 (US)
(74) Representative: Tunstall, Christopher Stephen

(56) References cited:
- US-A- 4 441 495
- US-A- 4 911 163
- US-A- 6 102 922

## Description

### Field of the Invention

The present invention has application in conventional endoscopic and open surgical instrumentation as well as application in robotic-assisted surgery. The present invention has even further relation to adjustable surgically implantable bands, such as gastric bands for the treatment of obesity.

### Background of the Invention

The percentage of the world's population suffering from morbid obesity is steadily increasing. Severely obese persons are susceptible to increased risk of heart disease, stroke, diabetes, pulmonary disease, and accidents. Because of the effect of morbid obesity to the life of the patient, methods of treating morbid obesity are being researched.

Numerous non-operative therapies for morbid obesity have been tried with virtually no permanent success. Dietary counseling, behavior modification, wiring a patient's jaws shut, and pharmacological methods have all been tried, and failed to correct the condition. Mechanical apparatuses for insertion into the body through non-surgical means, such as the use of gastric balloons to fill the stomach have also been employed in the treatment of the condition. Such devices cannot be employed over a long term, however, as they often cause severe irritation, necessitating their periodic removal and hence interruption of treatment. Thus, the medical community has evolved surgical approaches for treatment of morbid obesity.

Most surgical procedures for treatment of morbid obesity may generally be classified as either being directed toward the prevention of absorption of food (malabsorption), or restriction of stomach to make the patient feel full (gastric restriction) The most common malabsorption and gastric restriction technique is the gastric bypass. In variations of this technique, the stomach is horizontally divided into two isolated pouches, with the upper pouch having a small food capacity. The upper pouch is connected to the small intestine, or jejunum, through a small stoma, which restricts the processing of food by the greatly reduced useable stomach. Since food bypass much of the intestines, the amount of absorption of food is greatly reduced.

There are many disadvantages to the above procedure. Typically the above mentioned procedure is performed in an open surgical environment. Current minimally invasive techniques are difficult for surgeons to master, and have many additional drawbacks. Also, there is a high level of patient uneasiness with the idea of such a drastic procedure which is not easily reversible. In addition, all malabsorption techniques carry ongoing risks and side effects to the patient, including malnutrition and dumping syndrome.

Consequently, many patients and physicians prefer to undergo a gastric restriction procedure for the treatment of morbid obesity. One of the most common procedures involves the implantation of an adjustable gastric band. Examples of an adjustable gastric band can be found in U.S. Patents 4,592,339 issued to Kuzmak; RE 36176 issued to Kuzmak; 5,226,429 issued to Kuzmak; 6,102,922 issued to Jacobson and 5,601,604 issued to Vincent. In accordance with current practice, a gastric band is operatively placed to encircle the stomach. This divides the stomach into two parts with a stoma in-between. An upper portion, or a pouch, which is relatively small, and a lower portion which is relatively large. The small partitioned portion of the stomach effectively becomes the patients new stomach, requiring very little food to make the patient feel full.

Once positioned around the stomach, the ends of the gastric band are fastened to one another and the band is held securely in place by folding a portion of the gastric wall over the band and closing the folded tissue with sutures placed therethrough thereby preventing the band from slipping and the encircled stoma from expanding.

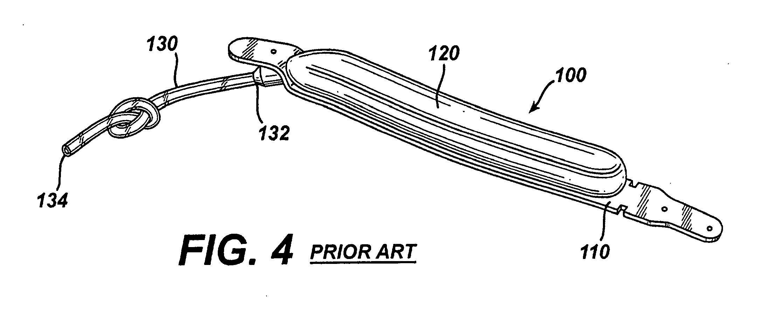

Figure 4 shows a prior art adjustable gastric band 100 such as those described in the above references. Gastric band 100 includes a flexible substantially non-extensible portion 110, and expandable fluid inflatable portion 120 attached thereto. Band 100 also includes a catheter tube 130 in fluid communication with inflatable portion 120. Catheter tube 130 has a distal end 132 attached to the inflatable portion 120 and a proximal end 134 extending therefrom.

The band typically arrives to the physician open to the ambient pressure and hence substantially filled with air. With the current devices, surgeons must typically leak test the band by filling them with air and immersing in saline. Additionally, because fluid adjustable bands are typically filled with saline or some other liquid, the physician must first evacuate the air from the band, close off the end of the device and then implant the device. Evacuation of air allows smooth insertion behind the stomach during the procedure. As seen from Figure 4, with prior art bands the physician would evacuate the band, typically with a syringe, and then tie a knot at the proximal end 134 to prevent air from entering the inflatable portion 120. After implantation, proximal end of the catheter tube is then attached to a fluid injection port and then the knot is untied. The inflatable portion is in fluid communication with a this remote injection site, or port.

Prior art bands requiring the tying of a knot in the catheter tube prior to implantation have some disadvantages. In tying the knot, the physician may damage the tube, or not tie the knot tight enough to prevent introduction of air. In addition, the physician may place the knot near to the catheter tube's proximal end, thereby not leaving enough room to attach it to a port. Typically the surgeon will then cut the tube on the side of the knot closest to the band before attaching to the port. If the knot is too close to the band, cutting it will not leave enough tube length to attach to the port. This situation necessitates the surgeon spending time to untie the knot. This can be difficult and time consuming because the tube is wet, the knot is tight to prevent air leakage and the surgeon has gloves on that make it difficult to untie the knot. A further disadvantage is that the band must be leak tested before implantation. This adds time and requires O.R. personnel to leak test the band.

### Summary of the Invention

In accordance with the present invention, there is provided a method of assembling a surgically implantable fluid adjustable device. The device has an elongated substantially air impermeable flexible inflatable portion, an elongated flexible and substantially inextensible band portion attached to the inflatable portion, and a catheter tube in fluid communication with the inflatable portion. The catheter tube has a distal end attached to the inflatable portion and an open proximal end extending therefrom. The method involves substantially evacuating the inflatable portion of fluids, and inserting a removable plug into the open proximal end of the catheter tube such that it substantially prevents introduction of air through the proximal end.

### Detailed Description of the Drawings

The novel features of the invention are set forth with particularity in the appended claims. The invention itself, however, both as to organization and methods of operation, together with further objects and advantages thereof, may best be understood by reference to the following description, taken in conjunction with the accompanying drawings in which:

Figure 1 is a perspective view of an surgically implantable fluid adjustable device 1 made in accordance with the present invention.

Figure 2 is a perspective view of a proximal end of a catheter tube and a plug made in accordance with the present invention.

Figure 3 is a cross section of the device shown in Figure 1, taken along lines 3-3.

Figure 4 is a perspective view of a prior art surgically implantable fluid adjustable device.

Figure 5 is a perspective view showing the band of figure 1 implanted around the stomach of a patient and attached to an injection port.

### Detailed Description of the Invention

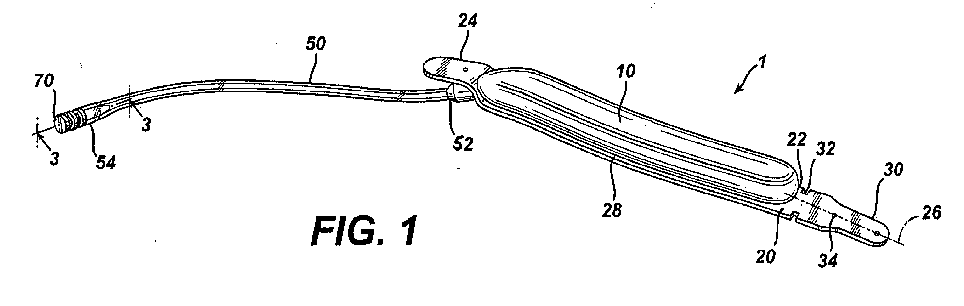

Referring now to Figure 1, there is shown a surgically implantable device 1 made in accordance with the present invention. The device includes an elongated flexible inflatable balloon portion 10. Balloon portion 10 is substantially evacuated of fluids prior to the devices implantation in a patient. Balloon portion 10 can be made from any number of materials known to those skilled in the art including silicone and polyurethane. In addition, such bands can be coated with materials to improve the prevention of diffusion. Such coatings include titanium powder and are described in PCT patent application WO 2004/010910 A1.

Device 1 further includes and an elongated flexible and substantially inextensible band portion 20. The band portion has a distal end 22, a proximal end 24 and a longitudinal axis 26 therebetween. Band portion 20 can be made from any number of materials known to those skilled in the art including silicone and polyurethane. The band portion is attached to the balloon portion along an inner face 28 of the band portion 20. The inflatable or balloon portion 10 can be attached to band portion 20 by any number of means known to those skilled in the art including using a silicone adhesive. The two portions may also be integrally manufactured as one part. In addition, band portion 20 can be formed to take on an undeployed curved shape as is described in U.S. Patent Application Serial No. 10/784,416 filed on February 20, 2004.

The distal and proximal ends of the band portions preferably include means for attaching such ends together. There are various means for attaching the distal and proximal ends of the band together. Many of these are described in co-pending and commonly assigned U.S. Patent Application Serial No. 60/483,353 filed September 30, 2003, 60/507,916 filed September 30, 2003 and 60/507,625 filed September 30, 2003. Figure 1 shows the distal end of the band 22 as comprising a tab 30 having notches 32. This tab 30 would be inserted into a slot (not shown) on the proximal end 24 of band 20. Tab 30 also includes suture holes 34 and 36, one of which would line up with suture hole 38 on the proximal end 24 of band 20. After the tab 30 is inserted into the slot, and the physician is pleased with the final position of the band, the ends 22 and 24 are then often sutured together to better secure the band in position. However, many alternative locking means, such as those described in the above reference, do not need to use suture.

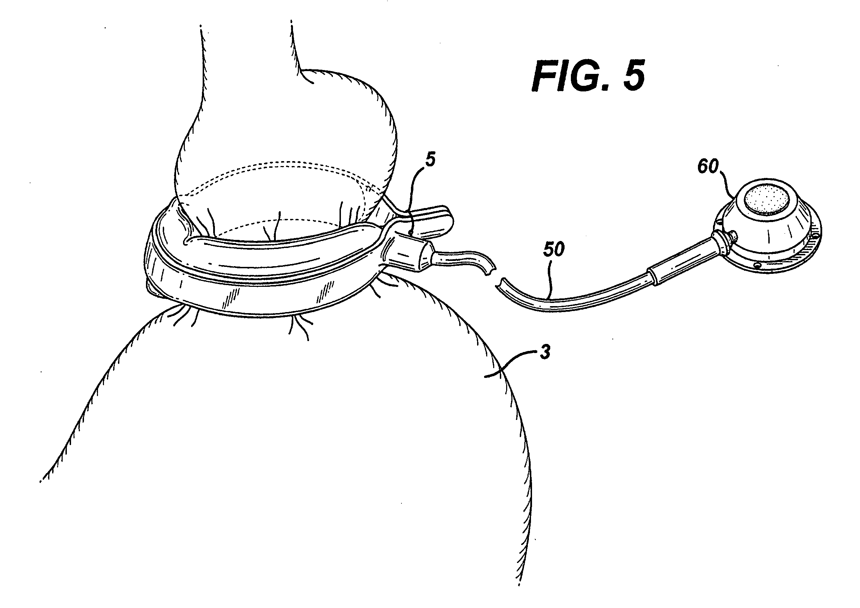

As seen in Figure 5, inflatable portion 10 is shown as being in fluid communication with an injection port 60 via a catheter tube 50. However, inflatable portion 10 could also be fluidly connected to an implanted reservoir such as those used with remotely controlled bands. Such a band is described in U.S. Patent 6,453,907 issued on September 24, 2002. Port 60 is of the type well known in the medical field not only for gastric bands, but such ports are also used for vascular access for drug delivery. After device 1 is implanted into a patient, port 60 is attached just below the skin of the patient, so that fluid can be inserted and withdrawn from the inflatable portion with a syringe. Catheter tube 50 can be integral with inflatable portion 10 or can be a separate piece.

Referring back to Figure 1, catheter tube 50 is in fluid communication with the inflatable portion 10. Tube 50 has a distal end 52 attached to the inflatable portion and an open proximal end 54 extending therefrom. As seen in the figures, the device 1 further includes a removable plug 70 inserted into the open proximal end of the catheter tube such that it substantially prevents introduction of air through the proximal end.

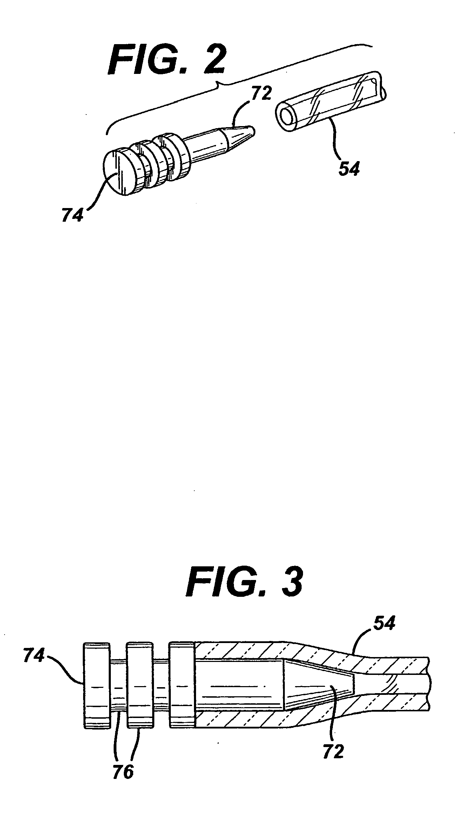

Plug 70 can better be understood by referring to Figures 2 and 3. Plug 70 has a distal end 72 and a proximal end 74. Plug 70 can be made from any number of polymeric or metallic materials know in the art including polycarbonate or stainless steel, and can be made by any number of methods known to those skilled in the art including injection molding, machining, etc. Distal end 72 is preferably slanted, as shown in the figures, so that the plug can be easily inserted during manufacture. Proximal end 74 preferably includes a gripping surface 76 so that the user can easily remove the plug. The gripping surface shown in the figures is a series of ridges and indentations but could take other forms known to those skilled in the art.

After the catheter tube 50, inflatable portion 10, and band portion 20 are assembled together, the manufacturer would then substantially evacuate the band of fluids using any number of known manufacturing techniques. Thereafter, plug 70 would be inserted into the proximal end of the catheter tube. The device 1 can then be inserted into a package and sent to the physician. Sometime prior to shipping the device, it should be sterilized using any number of known methods including electron beam (Ebeam), gamma radiation, or ethylene oxide.

Plug 70 substantially prevents air from entering into the catheter tube 50 and into the inflatable portion 10 prior to the band being implanted into a patient and the plug removed. This design does not require the physician to evacuate the band and tie a knot in the catheter tube prior to implantation. This reduces the risk that the tube will become damaged during the evacuation or knot tying steps. Plug 70 preferably has an outer diameter which is not substantially greater than the outer diameter of the distal end of the catheter tube containing the plug. This gives the plug a low profile so that the surgeon can grasp the plug and easily pull the band around the stomach with out the plug catching on the surrounding tissue. The band is evacuated of fluids and the plug placed therein during manufacture and shipped in that condition to the physician. An additional advantage of this invention is when the band is used with anti-diffusive coatings the band can be evacuated at the time of manufacture and then shipped to the surgeon in the evacuated condition. The surgeon may then bypass the entire step of leak testing the band because the collapsed condition of the balloon will be obvious to the surgeon and thereby confirm that the band has maintained an air-tight seal.

When implanting the band the physician would prepare the patient and the surgical site therein according to normal well known surgical procedures. The pre-evacuated band is inserted through a trocar. The surgeon has previously dissected the gastrophrenic ligament and the lesser curvature and created the retrogastric tunnel posterior to the stomach. The band is then grasped on the plug and pulled posteriorly through the retrogastric tunnel and then be placed around the organ, as shown in Figure 5, and secured with a suture 5 or the like. Then, the physician would implant the port 60, typically by securing it to the fascia below the skin, using normal well known surgical techniques. Thereafter, the physician would remove plug 70, and attach distal end 52 of the catheter tube to the injection port 60.

It will become readily apparent to those skilled in the art that the above invention has equally applicability to other types of implantable bands. For example, bands are used for the treatment of fecal incontinence. One such band is described in U.S. Patent 6,461,292. Bands can also be used to treat urinary incontinence. One such band is described in U.S. Patent Application 2003/0105385. Bands can also be used to treat heartburn and/or acid reflux. One such band is described in U.S. Patent 6,470,892. Bands can also be used to treat impotence. One such band is described in U.S. Patent Application 2003/0114729.

While preferred embodiments of the present invention have been shown and described herein, it will be obvious to those skilled in the art that such embodiments are provided by way of example only. Numerous variations, changes, and substitutions will now occur to those skilled in the art without departing from the invention. For example, as would be apparent to those skilled in the art, the disclosures herein have equal application in robotic-assisted surgery. In addition, it should be understood that every structure described above has a function and such structure can be referred to as a means for performing that function. Accordingly, it is intended that the invention be limited only by the scope of the appended claims.

## Claims

1. A method of assembling a surgically implantable fluid adjustable device (1) comprising:
a. providing a device (1) comprising an elongated substantially air impermeable flexible inflatable portion (10), an elongated flexible and substantially inextensible band portion (20) attached to said inflatable portion (10), and a catheter tube (50) in fluid communication with said inflatable portion (10), said catheter tube (50) having a distal end (52) attached to said inflatable portion (10) and an open proximal end (54) extending therefrom; and
b. substantially evacuating said elongated substantially air impermeable flexible inflatable portion (10) of fluids; and
**characterised by**
c. inserting a removable plug (70) into said open proximal end (54) of said catheter tube 950) such that it substantially prevents introduction of air through said proximal end (54).

2. The method of claim 1 further comprising:
d. inserting said device (1) into a package.

3. The method of claim 2 further comprising:
e. sterilizing said device (1).

## Patentansprüche

1. Verfahren zur Herstellung einer chirurgisch implantierbaren Fluid justierbaren Vorrichtung (1), wobei das Verfahren die folgenden Schritte aufweist:
a) Bereitstellen einer Vorrichtung (1) mit einem gestreckten im Wesentlichen luftundurchlässigen flexiblen aufblasbaren Bereich (10), einem gestreckten flexiblen und im Wesentlichen nicht dehnbaren Bandbereich (20), welcher an dem aufblasbaren Bereich (10) angeordnet ist und einem Kathetertubus (50), welcher in Fluidverbindung mit dem aufblasbaren Bereich (10) ist, wobei der Kathetertubus (50) ein an dem aufblasbaren Bereich (10) angeordnetes distales Ende (52) und ein offenes proximales Ende (54), welches sich davon erstreckt, aufweist; und
b) Evakuieren der Fluide des gestreckten im Wesentlichen luftundurchlässigen flexiblen aufblasbaren Bereiches (10); und
**gekennzeichnet durch**
c) Einführen eines lösbaren Verschlusses (70) in das offene proximale Ende (54) des Kathetertubus (50), so daß eine Einführung von Luft **durch** das proximale Ende (54) im Wesentlichen verhindert wird.

2. Verfahren nach Anspruch 1, **dadurch gekennzeichnet, daß** das Verfahren den folgenden Schritt aufweist:
d) Einführen der Vorrichtung (1) in eine Verpackung.

3. Verfahren nach Anspruch 2, **dadurch gekennzeichnet, daß** das Verfahren den folgenden Schritt aufweist:
e) Sterilisieren der Vorrichtung (1).

## Revendications

1. Méthode d'assemblage d'un dispositif implantable chirurgicalement réglable par liquide (1) comprenant :
a. la proposition d'un dispositif (1) comprenant une partie gonflable flexible et allongée substantiellement imperméable à l'air (10), une partie formant ruban flexible et allongé et substantiellement non extensible (20) fixée à ladite partie gonflable (10), et un tube de cathéter (50) en communication liquide avec ladite partie gonflable (10), ledit tube de cathéter (50) ayant une extrémité distale (52) fixée à ladite partie gonflable (10) et une extrémité proximale ouverte (54) s'étendant depuis celle-ci ; et
b. l'évacuation substantielle des liquides de ladite partie gonflable souple et allongée substantiellement imperméable à l'air (10), et
**caractérisé par**
c. l'insertion d'un bouchon amovible (70) dans ladite extrémité proximale ouverte (54) dudit tube de cathéter (50) de telle sorte qu'il empêche sensiblement l'introduction d'air par ladite extrémité proximale (54).

2. Méthode selon la revendication 1 comprenant en outre :
d. l'insertion dudit dispositif (1) dans un emballage.

3. Méthode selon la revendication 2 comprenant en outre :
e. la stérilisation dudit dispositif (1).
